# EUROPEAN PATENT APPLICATION

(11) **EP 2 357 253 A1**
(43) Date of publication of application: **17.08.2011**
(21) Application number: 10153395.8
(22) Date of filing: 12.02.2010
(51) Int. Cl.: C12Q 1/68

(54) **Method for genotyping blood samples for red blood cell and/or platelet surface antigens**

(71) Applicant: Oesterreichisches Rotes Kreuz, 1040 Wien (AT)
(72) Inventor: Jungbauer, Christof, 1120 Wien (AT)
(74) Representative: Bachinger-Fuchs, Eva-Maria

(57) **Abstract**

The invention relates to a method for genotyping blood samples for red blood cell and/or platelet surface antigens containing antigens of ≥99% prevalence and antigens of <99% prevalence in the population, the method comprising the steps of:
(a) extracting DNA from a blood sample,
(b) subjecting the DNA to multiplex PCR, targeting at least three red blood and/or platelet surface antigen genotypes in each reaction mix of at least two reaction mixes,
(c) subjecting amplification products to analysis by electrophoresis, and
(d) comparing the electrophoresis bands obtained in step (c) with bands of positive controls of the red blood cell and/or platelet surface antigens, with identical bands indicating the presence of the red blood cell and/or platelet surface antigens in the sample,

wherein in each PCR reaction mix targets of antigens of ≥99% and <99% prevalence are combined such that alternating bands of antigens of ≥99% prevalence and of <99% prevalence are obtained in step (c). The method is cost-efficient and allows easy interpretation of results.

SEQUENCE LISTING

<110>  Österreichisches Rotes Kreuz, Generalsekretariat

<120>  Method for genotyping blood samples for red blood cell and/or
       platelet surface antigens

<130>  Ö 12834

<160>  72

<170>  PatentIn version 3.3

<210>  1
<211>  20
<212>  DNA
<213>  Artificial

<220>
<223>  PCR primer for genotype MNS1

<400>  1
cagcatcaag taccactggt                                                   20


<210>  2
<211>  22
<212>  DNA
<213>  Artificial

<220>
<223>  reverse primer for genotype MNS1

<400>  2
ttcagaggca agaattcctc ca                                                22


<210>  3
<211>  21
<212>  DNA
<213>  Artificial

<220>
<223>  PCR primer for genotype MNS2

<400>  3
tcagcattaa gtaccactga g                                                 21


<210>  4
<211>  22
<212>  DNA
<213>  Artificial

<220>
<223>  reverse primer for genotype MNS2

<400>  4
ttcagaggca agaattcctc ca                                                22


<210>  5
<211>  19
<212>  DNA
<213>  Artificial

<220>
<223>  PCR primer for genotype MNS3

<400>  5
cgatggacaa gttgtccca                                                    19


<210>  6
<211>  21
<212>  DNA
<213>  Artificial

<220>
<223>  reverse primer for genotype MNS3

<400>  6
ggagtaatgg ctccatatgc c                                                 21


<210>  7
<211>  19
<212>  DNA
<213>  Artificial

<220>
<223>  PCR primer for genotype MNS4

<400>  7
cgatggacaa gttgtcccg                                                    19


<210>  8
<211>  21
<212>  DNA
<213>  Artificial

<220>
<223>  reverse primer for genotype MNS4

<400>  8
ggagtaatgg ctccatatgc c                                                 21


<210>  9
<211>  16
<212>  DNA
<213>  Artificial

<220>
<223>  PCR primer for genotype LU1

<400>  9
cgggagctcg cccgca                                                       16


<210>  10
<211>  21
<212>  DNA
<213>  Artificial

<220>
<223>  reverse primer for genotype LU1

<400>  10
gacagtgtcc ctttgttggg g                                                 21


<210>  11
<211>  16
<212>  DNA
<213>  Artificial

<220>
<223>  PCR primer for genotype LU2

<400>  11
cgggagctcg cccgcg                                                       16


<210>  12
<211>  21
<212>  DNA
<213>  Artificial

<220>
<223>  reverse primer for genotype LU2

<400>  12
gacagtgtcc ctttgttggg g                                                 21


<210>  13
<211>  19
<212>  DNA
<213>  Artificial

<220>
<223>  PCR primer for genotype LU8

<400>  13
ctctcccaga gggctacat                                                    19


<210>  14
<211>  20
<212>  DNA
<213>  Artificial

<220>
<223>  reverse primer for genotype LU8

<400>  14
gcttgtgcga ccaattgagg                                                   20


<210>  15
<211>  19
<212>  DNA
<213>  Artificial

<220>
<223>  PCR primer for genotype LU14

<400>  15
ctctcccaga gggctacaa                                                    19


<210>  16
<211>  20
<212>  DNA
<213>  Artificial

<220>
<223>  reverse primer for genotype LU14

<400>  16
gcttgtgcga ccaattgagg                                                   20


<210>  17
<211>  20

<212>  DNA
<213>  Artificial

<220>
<223>  PCR primer for genotype KEL1

<400>  17
actcatcaga agtctcagca                                                    20


<210>  18
<211>  21
<212>  DNA
<213>  Artificial

<220>
<223>  reverse primer for genotype KEL1

<400>  18
ctagagggtg ggtcttcttc c                                                  21


<210>  19
<211>  19
<212>  DNA
<213>  Artificial

<220>
<223>  PCR primer for genotype KEL2

<400>  19
ctcatcagaa gtctcagcg                                                     19


<210>  20
<211>  21
<212>  DNA
<213>  Artificial

<220>
<223>  reverse primer for genotype KEL2

<400>  20
ctagagggtg ggtcttcttc c                                                  21


<210>  21
<211>  21
<212>  DNA
<213>  Artificial

<220>
<223>  PCR primer for genotype KEL3

<400>  21
tgtcaatctc catcacttca t                                                  21


<210>  22
<211>  17
<212>  DNA
<213>  Artificial

<220>
<223>  reverse primer for genotype KEL3

<400>  22
ctgcccgcac aggtggc                                                       17

<210>  23
<211>  19
<212>  DNA
<213>  Artificial

<220>
<223>  PCR primer for genotype KEL4

<400>  23
caatctccat cacttcacg                                                    19


<210>  24
<211>  17
<212>  DNA
<213>  Artificial

<220>
<223>  reverse primer for genotype KEL4

<400>  24
ctgcccgcac aggtggc                                                      17


<210>  25
<211>  15
<212>  DNA
<213>  Artificial

<220>
<223>  PCR primer for genotype KEL6

<400>  25
gcctgggggc tgccc                                                        15


<210>  26
<211>  18
<212>  DNA
<213>  Artificial

<220>
<223>  reverse primer for genotype KEL6

<400>  26
cttgctcact ggttctgc                                                     18


<210>  27
<211>  11
<212>  DNA
<213>  Artificial

<220>
<223>  PCR primer for genotype KEL7

<400>  27
gggggctgcc t                                                            11


<210>  28
<211>  18
<212>  DNA
<213>  Artificial

<220>
<223>  reverse primer for genotype KEL7

<400>  28
cttgctcact ggttctgc                                                    18


<210>  29
<211>  17
<212>  DNA
<213>  Artificial

<220>
<223>  PCR primer for genotype KEL11

<400>  29
gagctggtcg atagtga                                                     17


<210>  30
<211>  20
<212>  DNA
<213>  Artificial

<220>
<223>  reverse primer for genotype KEL11

<400>  30
ctcttcttct catgcccctc                                                  20


<210>  31
<211>  16
<212>  DNA
<213>  Artificial

<220>
<223>  PCR primer for genotype KEL17

<400>  31
agctggtcga tagtgg                                                      16


<210>  32
<211>  20
<212>  DNA
<213>  Artificial

<220>
<223>  reverse primer for genotype KEL17

<400>  32
ctcttcttct catgcccctc                                                  20


<210>  33
<211>  19
<212>  DNA
<213>  Artificial

<220>
<223>  PCR primer for genotype KEL21

<400>  33
caatctccat cacttcaca                                                   19


<210>  34
<211>  17
<212>  DNA
<213>  Artificial

<220>
<223>  reverse primer for genotype KEL21

<400>  34
ctgcccgcac aggtggc                                                      17


<210>  35
<211>  21
<212>  DNA
<213>  Artificial

<220>
<223>  PCR primer for genotype FY1

<400>  35
cagctgcttc caggttggga c                                                 21


<210>  36
<211>  22
<212>  DNA
<213>  Artificial

<220>
<223>  reverse primer for genotype FY1

<400>  36
ctcattagtc cttggctctt at                                                22


<210>  37
<211>  21
<212>  DNA
<213>  Artificial

<220>
<223>  PCR primer for genotype FY2

<400>  37
cagctgcttc caggttggga t                                                 21


<210>  38
<211>  21
<212>  DNA
<213>  Artificial

<220>
<223>  reverse primer for genotype FY2

<400>  38
tcattagtcc ttggctctta t                                                 21


<210>  39
<211>  22
<212>  DNA
<213>  Artificial

<220>
<223>  PCR primer for genotype FY0

<400>  39
ctcattagtc cttggctctt ac                                                22


<210>  40
<211>  21

<212>  DNA
<213>  Artificial

<220>
<223>  reverse primer for genotype FY0

<400>  40
cagctgcttc caggttggga t                                                21


<210>  41
<211>  20
<212>  DNA
<213>  Artificial

<220>
<223>  PCR primer for genotype FYX

<400>  41
gcttttcaga cctctcttct                                                  20


<210>  42
<211>  18
<212>  DNA
<213>  Artificial

<220>
<223>  reverse primer for genotype FYX

<400>  42
caaattccca cagtgagc                                                    18


<210>  43
<211>  20
<212>  DNA
<213>  Artificial

<220>
<223>  PCR primer for genotype FY5

<400>  43
gcttttcaga cctctcttcc                                                  20


<210>  44
<211>  18
<212>  DNA
<213>  Artificial

<220>
<223>  reverse primer for genotype FY5

<400>  44
caaattccca cagtgagc                                                    18


<210>  45
<211>  21
<212>  DNA
<213>  Artificial

<220>
<223>  PCR primer for genotype JK1

<400>  45
gtctttcagc cccatttgcg g                                                21

<210> 46
<211> 20
<212> DNA
<213> Artificial

<220>
<223> reverse primer for genotype JK1

<400> 46
ccaaggccaa gtgtcagtgc 20


<210> 47
<211> 22
<212> DNA
<213> Artificial

<220>
<223> PCR primer for genotype JK2

<400> 47
agtctttcag ccccatttgc ga 22


<210> 48
<211> 20
<212> DNA
<213> Artificial

<220>
<223> reverse primer for genotype JK2

<400> 48
ccaaggccaa gtgtcagtgc 20


<210> 49
<211> 15
<212> DNA
<213> Artificial

<220>
<223> PCR primer for genotype DI1

<400> 49
gggccaggga ggcca 15


<210> 50
<211> 19
<212> DNA
<213> Artificial

<220>
<223> reverse primer for genotype DI1

<400> 50
tggctccata tggtgcctg 19


<210> 51
<211> 12
<212> DNA
<213> Artificial

<220>
<223> PCR primer for genotype DI2

<400>  51
ccagggaggc cg                                                          12


<210>  52
<211>  19
<212>  DNA
<213>  Artificial

<220>
<223>  reverse primer for genotype DI2

<400>  52
tggctccata tggtgcctg                                                   19


<210>  53
<211>  21
<212>  DNA
<213>  Artificial

<220>
<223>  PCR primer for genotype DI3

<400>  53
catcatccag atgggaaact t                                                21


<210>  54
<211>  20
<212>  DNA
<213>  Artificial

<220>
<223>  reverse primer for genotype DI3

<400>  54
ggactacctc taccccatcc                                                  20


<210>  55
<211>  20
<212>  DNA
<213>  Artificial

<220>
<223>  PCR primer for genotype DI4

<400>  55
atcatccaga tgggaaactc                                                  20


<210>  56
<211>  20
<212>  DNA
<213>  Artificial

<220>
<223>  reverse primer for genotype DI4

<400>  56
ggactacctc taccccatcc                                                  20


<210>  57
<211>  22
<212>  DNA
<213>  Artificial

<220>
<223>  PCR primer for genotype YT1

<400>  57
ctcatcaacg cgggagacta cc                                                22


<210>  58
<211>  21
<212>  DNA
<213>  Artificial

<220>
<223>  reverse primer for genotype YT1

<400>  58
gagccagaga gatgaacagt t                                                 21


<210>  59
<211>  22
<212>  DNA
<213>  Artificial

<220>
<223>  PCR primer for genotype YT2

<400>  59
ctcatcaacg cgggagacta ca                                                22


<210>  60
<211>  21
<212>  DNA
<213>  Artificial

<220>
<223>  reverse primer for genotype YT2

<400>  60
gagccagaga gatgaacagt t                                                 21


<210>  61
<211>  21
<212>  DNA
<213>  Artificial

<220>
<223>  PCR primer for genotype DO1

<400>  61
attcgatttg gccaattcct t                                                 21


<210>  62
<211>  21
<212>  DNA
<213>  Artificial

<220>
<223>  reverse primer for genotype DO1

<400>  62
tgacctcaac tgcaaccagt t                                                 21


<210>  63
<211>  21

<212>  DNA
<213>  Artificial

<220>
<223>  PCR primer for genotype DO2

<400>  63
attcgatttg gccaattcct c                                                21


<210>  64
<211>  20
<212>  DNA
<213>  Artificial

<220>
<223>  reverse primer for genotype DO2

<400>  64
gacctcaact gcaaccagtc                                                  20


<210>  65
<211>  15
<212>  DNA
<213>  Artificial

<220>
<223>  PCR primer for genotype CO1

<400>  65
gaacaaccag acggc                                                       15


<210>  66
<211>  18
<212>  DNA
<213>  Artificial

<220>
<223>  reverse primer for genotype CO1

<400>  66
ctgagaggat ggcggtgg                                                    18


<210>  67
<211>  17
<212>  DNA
<213>  Artificial

<220>
<223>  PCR primer for genotype CO2

<400>  67
gggaacaacc agacggt                                                     17


<210>  68
<211>  18
<212>  DNA
<213>  Artificial

<220>
<223>  reverse primer for genotype CO2

<400>  68
ctgagaggat ggcggtgg                                                    18

<210>  69
<211>  19
<212>  DNA
<213>  Artificial

<220>
<223>  PCR primer for genotype IN1

<400>  69
gtcgctacag catctctcc                                                    19


<210>  70
<211>  23
<212>  DNA
<213>  Artificial

<220>
<223>  reverse primer for genotype IN1

<400>  70
ccattcagct gtgggaaagg agc                                               23


<210>  71
<211>  17
<212>  DNA
<213>  Artificial

<220>
<223>  PCR primer for genotype IN2

<400>  71
cgctacagca tctctcg                                                      17


<210>  72
<211>  23
<212>  DNA
<213>  Artificial

<220>
<223>  reverse primer for genotype IN2

<400>  72
ccattcagct gtgggaaagg agc                                               23

## Description

The invention relates to a method for genotyping blood samples for red blood cell and/or platelet surface antigens containing antigens of ≥99% prevalence and antigens of <99% prevalence in the population, as well as to a multiplex PCR kit comprising primer pairs for amplifying the red blood cell and/or platelet surface antigens.

About 2% of the inhabitants of Europe and North America are carriers of alloantibodies to red cell antigens (Hoeltge GA, Arch Pathol Lab Med 1995). These alloantibodies mostly where derived by former transfusions or pregnancies. The prevalence of new antibody production during pregnancy is approximately 0,24% (Heddle NM, Transfusion 1994). In pretransfused patients the prevalence is approximately five percent. The rate of alloimmunization and the number of antibody specificities involved correlate with the number of transfusion events in the past. People who most repeatedly need transfusions are patients with hematological disorders. In adult patients with sickle cell anemia the alloimmunization rate was described to be 47% (Aygun B, Transfusion 2002). In Thalassemia major patients an alloantibody-prevalence of about 30% was seen (Ameen R.Transfusion. 2003; Castro O, Sandler SG Houston-Yu P, Rana S. Predicting the effect of transfusing only phenotype-matched RBCs to patients with sickle cell disease: theoretical and practical implications. Transfusion 2002 Jun;42(6):684-90.)

The blood supply for carriers of red blood cell (RBC) antibodies with antigen negative blood is one of the main tasks for every blood center. The effort of finding compatible blood with regard to RBC surface antigens is constrained by the prevalence of the antigen and the number of antibody specificities that have to be included in the search. Similar problems also apply to platelet surface antigens.

Antibodies to high frequency antigens (HFA), i.e. antigens with ≥99% prevalence in the population, represent a special problem. Hundreds to thousands of samples have to be screened to identify one appropriate donor. It was demonstrated that approximately one third of patients with HFA-antibodies does not get adequate supply with blood units needed (Seltsam A).

To be able to provide compatible blood units within short term blood centers usually type a subset of donors for further relevant antigens beside ABO, RhD and Kell. This "extended" typing in practice is limited by availability and costs of serological reagents. Partly, monoclonal antibodies enable continuous serological phenotyping. But for several clinically significant antigens only insufficient amounts of regulated polyclonal sera can be purchased.

As several milliliters of reagent are needed to find one HFA-negative donor, blood centers often use unregulated patient sera for screening and regulated reagents for confirmation of the results. Again, this procedure is limited by the existence of patients with a certain antibody specificity, the reaction strength of the antibody and the ABO antibodies in the patient serum.

In principle, all RBC antigens whose cause is known on DNA-level are accessible for genotyping. By now this applies to most RBC antigens. Compared to serology, in molecular biology, there is no shortage of reagents and, furthermore, regardless of the antigen, reaction mixes cost the same.

Different working groups have recently demonstrated the feasibility of several technical methods and platforms in high throughput genotyping of RBC antigens. All methods described combine attributes that are beneficial and others that are disadvantageous. For example, microarray applications enable simultaneous testing of thousands of polymorphisms. This advantage is balanced by the fact that these tests are still excessively costly.

C. Jungbauer (Transfus Med Hemother 2009;36:213-218) has described a multiplex PCR screening assay for 36 RBC antigens including 12 HFAs, wherein the assay consisted of six reaction mixes for all 36 genotypes for one blood donor and up to seven genotypes multiplexed per reaction mix.

To resolve the limitations that are immanently bound to serology, new approaches for extended antigen typing and the identification of HFA-negative blood donors are necessary.

The object of the invention, therefore, is to provide a method for genotyping blood samples for red blood cell and/or platelet surface antigens which provides more independence from availability of serological reagents. Moreover, the method should be scaleable and enable high throughput applications. Compared to serological methods, a higher cost efficiency should be guaranteed. In addition, the method should lead to results that can be easily interpreted.

This object is achieved by a method for genotyping blood samples for red blood cell and/or platelet surface antigens containing antigens of ≥99% prevalence and antigens of <99% prevalence in the population, the method comprising the steps of:
(a) extracting DNA from a blood sample,
(b) subjecting the DNA to multiplex PCR, targeting at least three red blood and/or platelet surface antigen genotypes in each reaction mix of at least two reaction mixes,
(c) subjecting amplification products to analysis by electrophoresis, and
(d) comparing the electrophoresis bands obtained in step (c) with bands of positive controls of the red blood cell and/or platelet surface antigens, with identical bands indicating the presence of the red blood cell and/or platelet surface antigens in the sample,
wherein in each PCR reaction mix targets of antigens of ≥99% and <99% prevalence are combined such that alternating bands of antigens of ≥99% prevalence and of <99% prevalence are obtained in step (c).

By combining one or more HFA related genotypes (RBC and platelet surface antigens of ≥99% prevalence) with appropriate genotypes of lower frequency (antigens of <99% prevalence) in one reaction mix the results on the electrophoresis gel, which are otherwise often diffuse and hard to differentiate, are clearly laid out and can easily be interpreted, due to the alternating bands of high and low frequency antigens. Furthermore, the high frequency antigens can be used as internal controls.

According to a preferred embodiment of the inventive method, at least 5, preferably 5, red blood and/or platelet surface antigen genotypes are targeted in each reaction mix in step (c).

Preferably, agarose gel electrophoresis or capillary electrophoresis is used in step (d).

In the method according to the present invention, it is preferred that the red blood cell and/or platelet surface antigens are selected from MNS1, MNS2, MNS3, MNS4, LU1, LU2, LU8, LU14, KEL1, KEL2, KEL3, KEL4, KEL6, KEL7, KEL11, KEL17, KEL21, FY1, FY2, FY0, FYX, FY5, JK1, JK2, DI1, DI2, DI3, DI4, YT1, YT2, DO1, DO2, CO1, CO2, IN1, IN2, LW5, LW6, RHCw, RHC, RHc, RHD, RHE, RHe, CROM1, CROM2, CROM5, CROM6, KN1, KN3, KN4, GE2 YUS, GE3 GE, GE leach PL, GE leach LN, C4A 3258A, C4A (3567A), C4A (3670C), C4B 3258G, C4B (3567G), C4B 3670G, LE1 200T, LE2 428G, LE2 571C, HPA-10a, HPA-10b, HPA-2a, HPA-2b, HPA-8a, HPA-8b, HPA-4a, HPA-4b, HPA-7a, HPA-7b, HPA-14a, HPA-14b, HPA-3a, HPA-3b, HPA-1a, HPA-1b, HPA-6b, HPA-15a, HPA-15b, HPA-11a, HPA-11b, HPA-13a, HPA-13b, HPA-17a, HPA-17b, HPA-5a, HPA-5b, HPA-16a and HPA-16b.

A preferred embodiment of the invention is characterized in that only red blood cell surface antigens are targeted in step (c).

Another embodiment is characterized in that only platelet surface antigens are targeted in step (c).

According to an embodiment, the at least two reaction mixes are selected from:
Mix 1: KEL21, DI4, DI1, LU2, FYX, IN2
Mix 2: FY0, DI3, DI2, LU1, FY5, IN1
Mix 3: KEL11, KEL6, LU8, YT2, CO1
Mix 4: KEL17, KEL7, LU14, YT1, CO2
Mix 5: FY1, KEL4, JK1, MNS4, KEL1, MNS1, DO1
Mix 6: FY2, KEL3, JK2, MNS3, KEL2, MNS2, DO2
Mix 7: KEL4, KEL1, LW5, RHCw, IN2, RHD
Mix 8: KEL3, CROM1, LW6, KEL2, IN1
Mix 9: GE leach PL, JK1, CROM6, RHC, KN1
Mix 10: GE leach LN, JK2, C4A (3567A), CROM5, RHc, KN3
Mix 11: LE1 200T, C4B 3670G, CROM2, RHE, KN4
Mix 12: LE2 571C, C4A (3670C), C4B (3567G), RHe
Mix 13: FY1, KEL11, KEL6, LU8, YT2, CO1
Mix 14: FY2, KEL17, KEL7, LU14, YT1, CO2
Mix 15: LE2 428G, KEL21, DI4, DI1, LU2, FYX, DO1
Mix 16: FY0, DI3, DI2, LU1, FY5, DO2
Mix 17: GE2 YUS, MNS4, C4A 3258A, MNS1
Mix 18: GE3 GE, MNS3, C4B 3258G, MNS2

Preferably, the multiplex PCR is performed using one or more of the following primer pairs: SEQ ID NO:1 and SEQ ID NO:2; SEQ ID NO:3 and SEQ ID NO:4; SEQ ID NO:5 and SEQ ID NO:6; SEQ ID NO:7 and SEQ ID NO:8; SEQ ID NO:9 and SEQ ID NO:10; SEQ ID NO:11 and SEQ ID NO:12; SEQ ID NO:13 and SEQ ID NO:14; SEQ ID NO:15 and SEQ ID NO:16; SEQ ID NO:17 and SEQ ID NO:18; SEQ ID NO:19 and SEQ ID NO:20; SEQ ID NO:21 and SEQ ID NO:22; SEQ ID NO:23 and SEQ ID NO:24; SEQ ID NO:25 and SEQ ID NO:26; SEQ ID NO:27 and SEQ ID NO:28; SEQ ID NO:29 and SEQ ID NO:30; SEQ ID NO:31 and SEQ ID NO:32; SEQ ID NO:33 and SEQ ID NO:34; SEQ ID NO:35 and SEQ ID NO:36, SEQ ID NO:37 and SEQ ID NO:38; SEQ ID NO:39 and SEQ ID NO:40; SEQ ID NO:41 and SEQ ID NO:42; SEQ ID NO:43 and SEQ ID NO:44; SEQ ID NO:45 and SEQ ID NO:46; SEQ ID NO:47 and SEQ ID NO:48; SEQ ID NO:49 and SEQ ID NO:50; SEQ ID NO:51 and SEQ ID NO:52; SEQ ID NO:53 and SEQ ID NO:54; SEQ ID NO:55 and SEQ ID NO:56; SEQ ID NO:57 and SEQ ID NO:58; SEQ ID NO:59 and SEQ ID NO:60; SEQ ID NO:61 and SEQ ID NO:62; SEQ ID NO:63 and SEQ ID NO:64; SEQ ID NO:65 and SEQ ID NO:66; SEQ ID NO:67 and SEQ ID NO:68; SEQ ID NO:69 and SEQ ID NO:70; and/or SEQ ID NO:71 and SEQ ID NO:72.

According to another embodiment, the at least two reaction mixes are selected from:
Mix 1: HPA-10b, HPA-2a, HPA-8b, HPA-4a, HPA-7b
Mix 2: HPA-10a, HPA-2b, HPA-8a, HPA-4b, HPA-7a
Mix 3: HPA-14b, HPA-3a, HPA-1a, HPA-6b
Mix 4: HPA-14a, HPA-3b, HPA-1b
Mix 5: HPA-14b, HPA-15a, HPA-11b, HPA-4a
Mix 6: HPA-14a, HPA-15b, HPA-11a, HPA-4b
Mix 7: HPA-13b, HPA-2a, HPA-17b, HPA-5a, HPA-16b
Mix 8: HPA-13a, HPA-2b, HPA-17a, HPA-5b, HPA-16a

In a further aspect, the present invention provides a multiplex PCR kit, comprising primer pairs for amplifying red blood cell and/or platelet surface antigens selected from MNS1, MNS2, MNS3, MNS4, LU1, LU2, LU8, LU14, KEL1, KEL2, KEL3, KEL4, KEL6, KEL7, KEL11, KEL 17, KEL21, FY1, FY2, FY0, FYX, FY5, JK1, JK2, DI1, DI2, DI3, DI4, YT 1, YT2, DO1, DO2, CO1, CO2, IN1, IN2, HPA-10a, HPA-10b, HPA-2a, HPA-2b, HPA-8a, HPA-8b, HPA-4a, HPA-4b, HPA-7a, HPA-7b, HPA-14a, HPA-14b, HPA-3a, HPA-3b, HPA-1a, HPA-1b, HPA-6b, HPA-15a, HPA-15b, HPA-11a, HPA-11b, HPA-13a, HPA-13b, HPA-17a, HPA-17b, HPA-5a, HPA-5b, HPA-16a and HPA-16b, wherein SEQ ID NO:1 and SEQ ID NO:2; SEQ ID NO:3 and SEQ ID NO:4; SEQ ID NO:5 and SEQ ID NO:6; SEQ ID NO:7 and SEQ ID NO:8; SEQ ID NO:9 and SEQ ID NO:10; SEQ ID NO:11 and SEQ ID NO:12; SEQ ID NO:13 and SEQ ID NO:14; SEQ ID NO:15 and SEQ ID NO:16; SEQ ID NO:17 and SEQ ID NO:18; SEQ ID NO:19 and SEQ ID NO:20; SEQ ID NO:21 and SEQ ID NO:22; SEQ ID NO:23 and SEQ ID NO:24; SEQ ID NO:25 and SEQ ID NO:26; SEQ ID NO:27 and SEQ ID NO:28; SEQ ID NO:29 and SEQ ID NO:30; SEQ ID NO:31 and SEQ ID NO:32; SEQ ID NO:33 and SEQ ID NO:34; SEQ ID NO:35 and SEQ ID NO:36, SEQ ID NO:37 and SEQ ID NO:38; SEQ ID NO:39 and SEQ ID NO:40; SEQ ID NO:41 and SEQ ID NO:42; SEQ ID NO:43 and SEQ ID NO:44; SEQ ID NO:45 and SEQ ID NO:46; SEQ ID NO:47 and SEQ ID NO:48; SEQ ID NO:49 and SEQ ID NO:50; SEQ ID NO:51 and SEQ ID NO:52; SEQ ID NO:53 and SEQ ID NO:54; SEQ ID NO:55 and SEQ ID NO:56; SEQ ID NO:57 and SEQ ID NO:58; SEQ ID NO:59 and SEQ ID NO:60; SEQ ID NO:61 and SEQ ID NO:62; SEQ ID NO:63 and SEQ ID NO:64; SEQ ID NO:65 and SEQ ID NO:66; SEQ ID NO:67 and SEQ ID NO:68; SEQ ID NO:69 and SEQ ID NO:70; and/or SEQ ID NO:71 and SEQ ID NO:72 are used as said primer pairs.

The multiplex PCR kit preferably further comprises a buffer, DNA polymerase, positive and/or negative controls.

A further advantage of the PCR screening method according to the invention is identifying suitable panel cells for serological antibody differentiation. Particularly HFA negative or low frequency antigen (LFA) positive RBCs are useful for reference centers for immunohematology to clarify uncommon antibody specificities.

For patients who repeatedly require transfusions an extended matching of antigens exceeding ABO, RhD, Kell, RhCcEe and any alloantibody present can be used to prevent further alloimmunization.

In patient testing the inventive multiplex-PCR assay can be used as a complement test to indicate HFA negative individuals in patients with alloantibodies to HFAs. Furthermore, it may be used in alloimmunized patients with positive direct agglutination test (DAT) if a certain specificity is assumed.

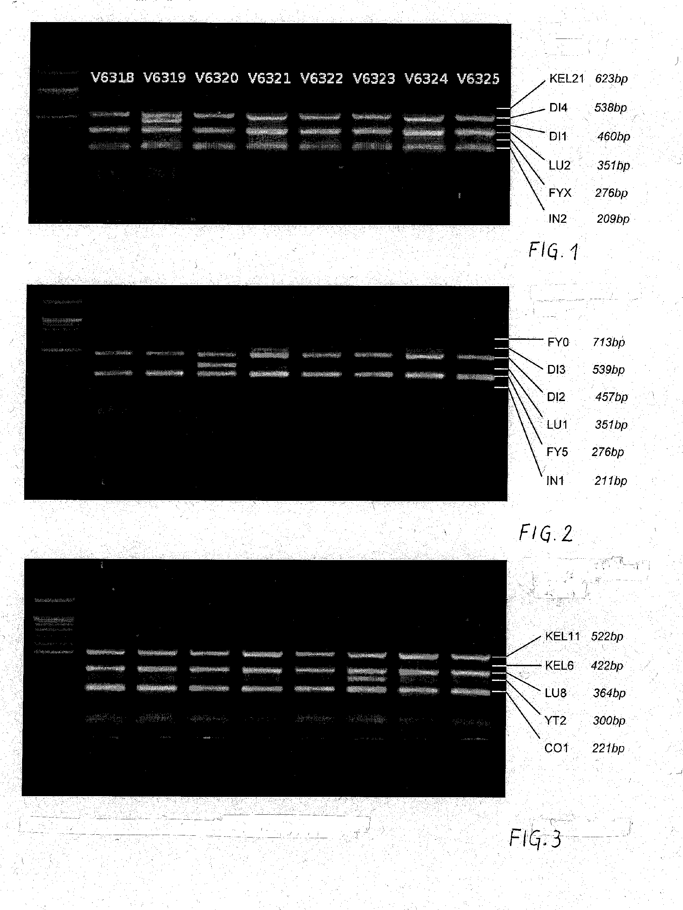

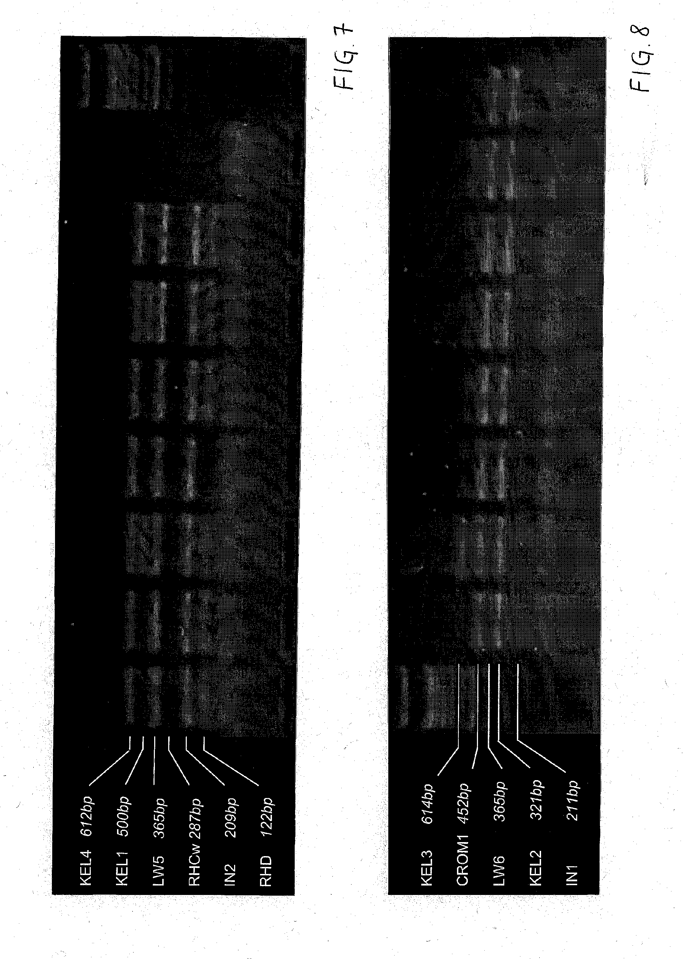

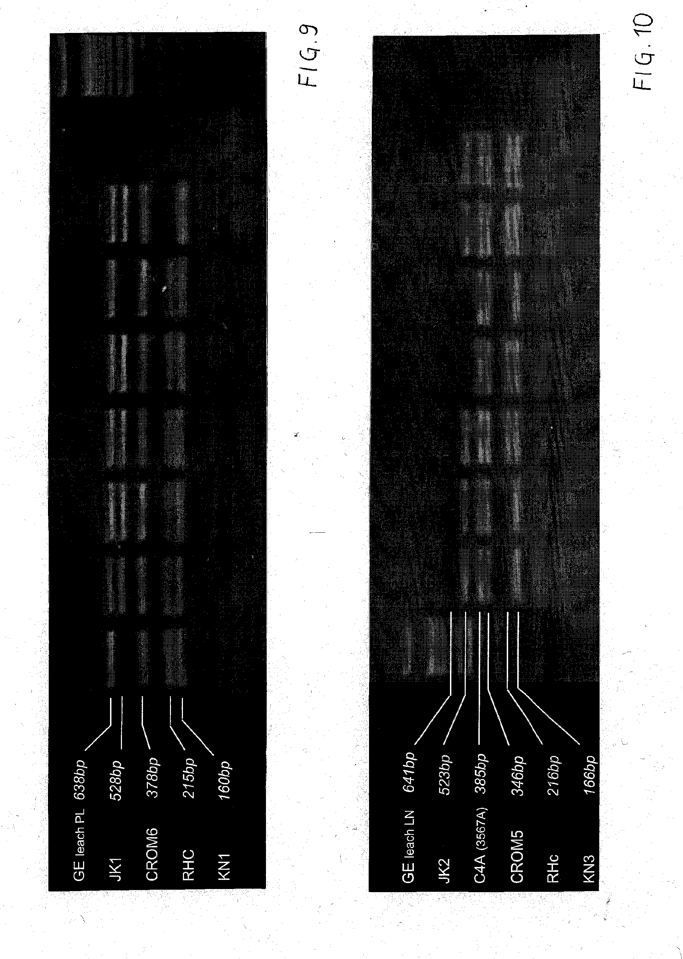

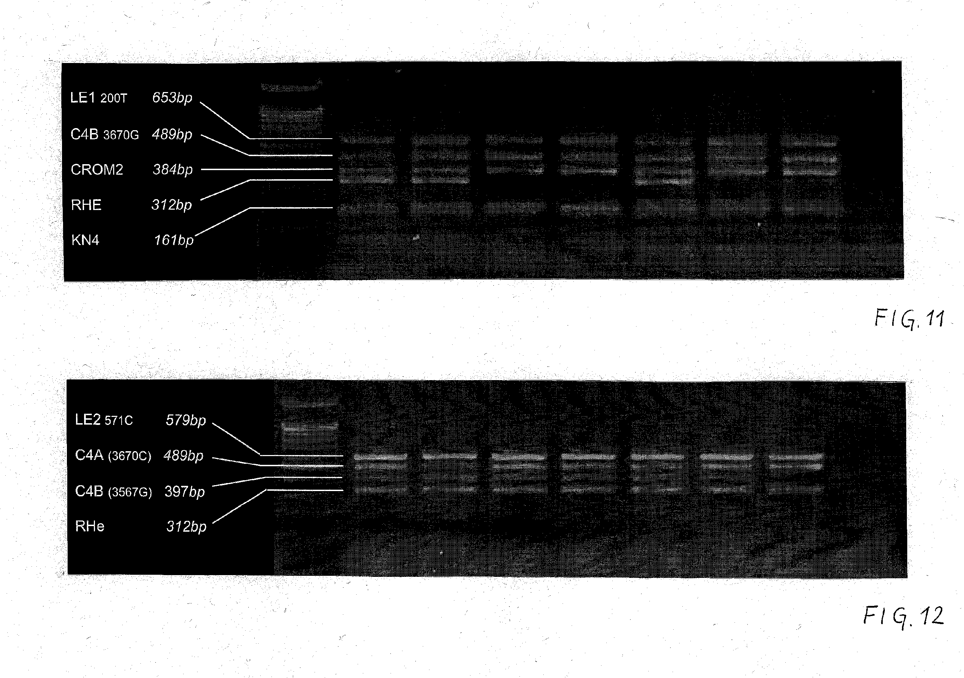

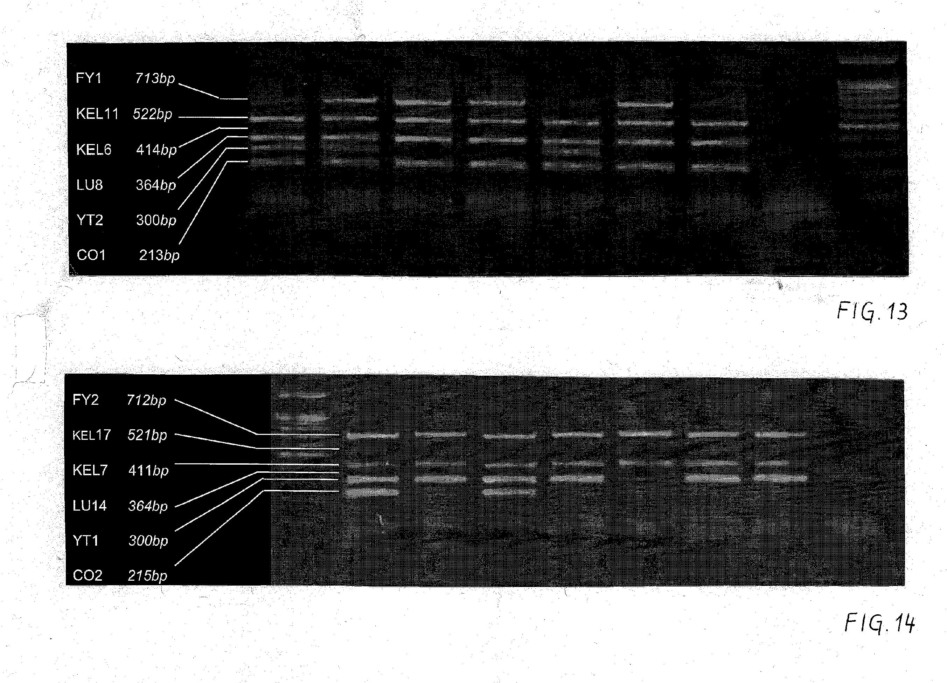

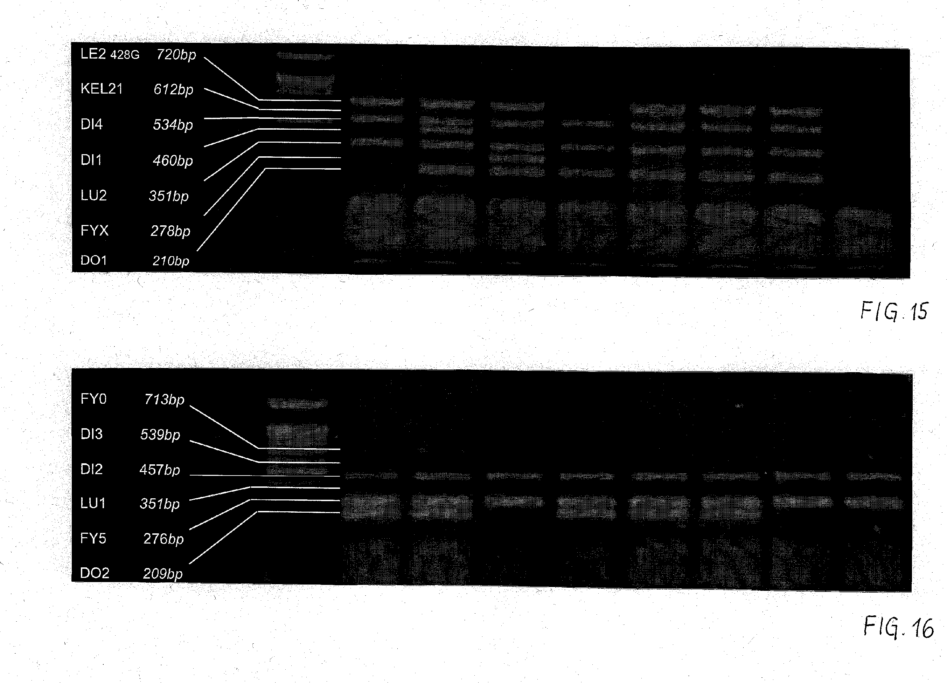

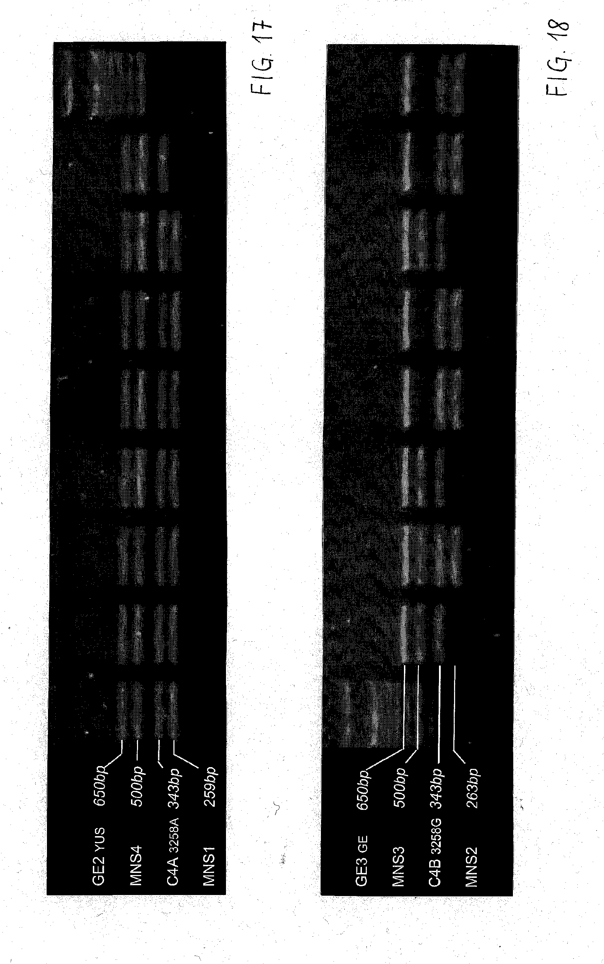

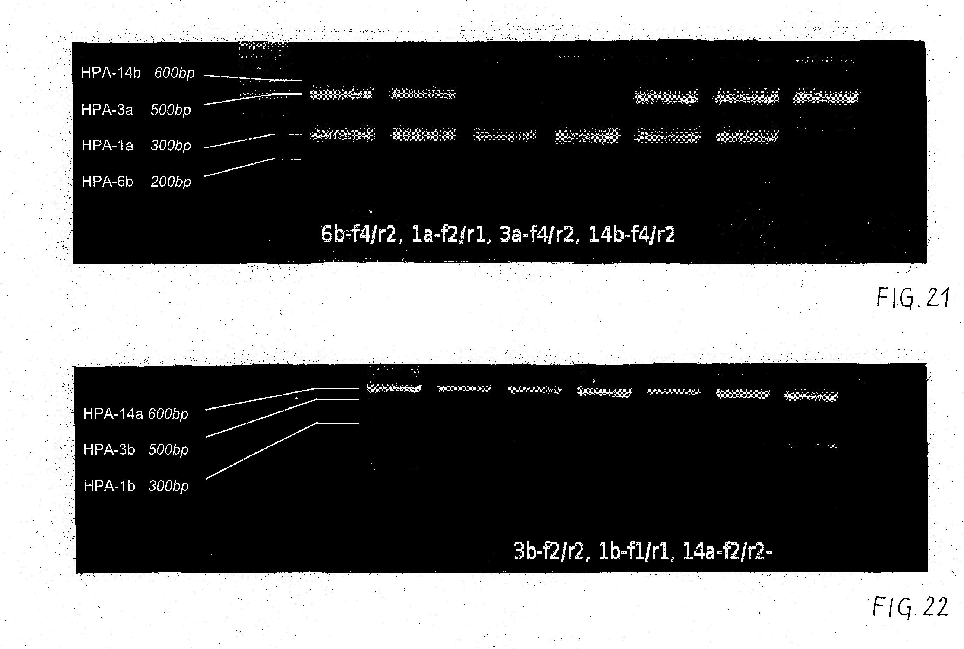

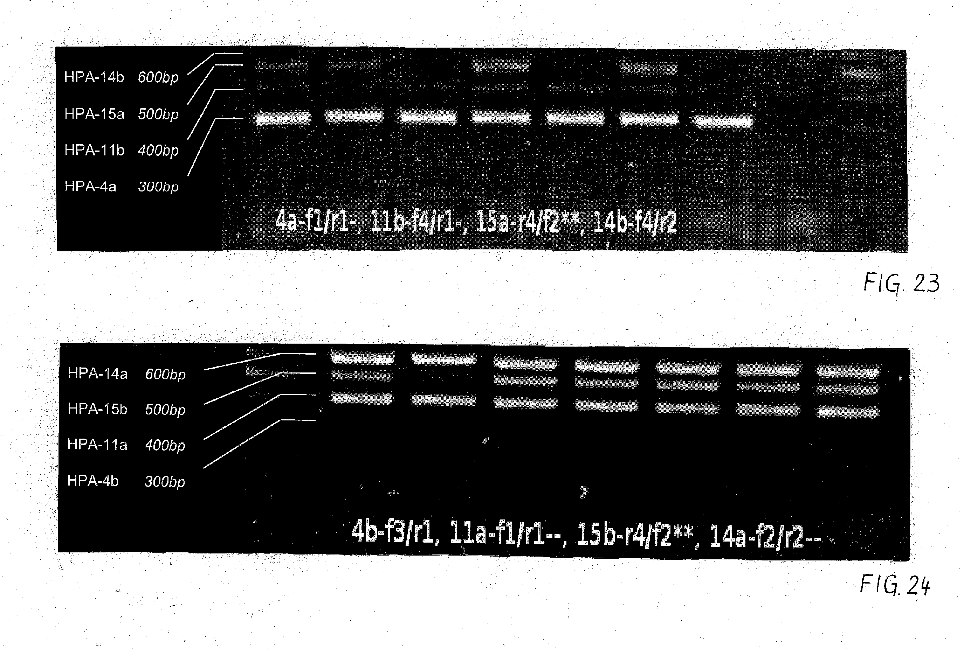

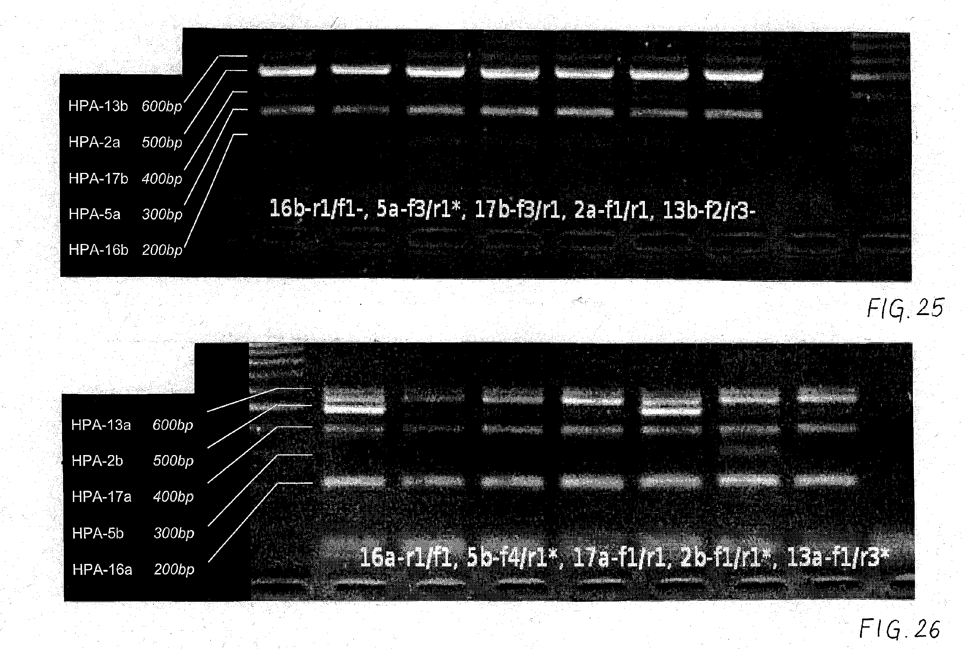

In the following, the invention will be described in more detail by way of examples and figures, wherein Fig. 1-26 illustrate the results of agarose gel electrophoresis analysis for several reaction mixes.

### Multiplex-PCR Assay

The selection of the antigens to be included in the multiplex-PCR assay was based on the prevalence of the alloantibody. Rh-antigens were not included in the assay because many blood centers routinely perform serological testing for RhD, C, c, E and e. Another aspect, especially for selection of appropriate HFAs, was the clinical significance of the alloantibody and its ability to cause hemolytic transfusion reactions.

### Example 1:

Oligonucleotide design and assortment of the critical genetic polymorphisms was performed using the NCBI dbRBC "The Blood Group Antigen Gene Mutation Database" (http://www.ncbi.nlm.nih.gov/projects/mhc/xslcgi.fcgi?cmd=bgmut/home) and the NCBI nucleotide database (http://www.ncbi.nih.gov/sites/entrez) both hosted by the National Center for Biotechnology Information (NCBI).

Particular genotypes causal for the following antigens were included in the PCR assay: in the MNS blood group system the genotypes for MNS1 ("M"), MNS2 ("N"), MNS3 ("S") and MNS4 ("s"); in the Lutheran system LU1 ("Lu^{a}"), LU2 ("Lu^{b}"), LU8 and LU14; in the Kell system KEL1 ("K"), KEL2 ("k"), KEL3 ("Kp^{a}"), KEL4 ("Kp^{b}"), KEL6 ("Js^{a}"), KEL7 ("Js^{b}"), KEL 11, KEL 17 and KEL21; in the Duffy system FY1 ("Fy^{a}"), FY2 ("Fy^{b}"), one FyX allele, Fy0 allele (-33 promoter silencing polymorphism) and Fy5 allele; in the Kidd system JK1 ("Jk^{a}") and JK2 ("Jk^{b}"); in the Diego system DI1 ("Di^{a}"), DI2 (Di^{b}"), DI3 ("Wr^{a}") and DI4 ("Wr^{b}"); in the YT ("Cartwright") system YT1 ("Yt^{a}") and YT2 ("Yt^{b}"); in the Dombrock system DO1 ("Do^{a}") and DO2 ("Do^{b}"); in the Colton system CO1 ("Co^{a}") and CO2 ("Co^{b}") and the IN1 ("In^{a}") and IN2 ("In^{b}") polymorphisms of the Indian blood group system.

The test system was developed using pretyped positive and negative controls. Most controls were serologically typed donors or patients of the Austrian Red Cross, Vienna blood center. Other controls for rare phenotypes were kindly supplied by some other centers. For some genotypes no controls were available.

For validation of the multiplex-PCR-assay DNA was extracted from 371 randomly selected blood samples. The blood was serologically phenotyped for 13 antigens where regulated reagents were available. Additionally, special samples with known phenotypes were included, such as, e.g., 99 KEL2 negative samples.

Subsequently, 6000 donors were tested with the multiplex-PCR assay. The aim was to identify new HFA negative donors and to add the antigen typing results to the existing database. The screening character of genotyping data was carefully documented. Screening results were discriminated from the data from phenotyping processed with certified reagents.

### Materials and Methods

### Genomic DNA samples

Ethylenediaminetetraacetate (EDTA)-anticoagulated blood samples or buffy coats of donors or patients were obtained subsequent to the routine testing. Genomic DNA extraction was performed using a Tecan RSP150 platform and QIAamp DNA Blood BioRobot 9604 Kit (Qiagen, Hilden, Germany). Alternatively, DNA was extracted manually with QIAamp DNA Blood Mini Kit (Qiagen, Hilden, Germany). DNA concentration in eluate was 34.8 µg/µl (+/-25,2; n=20) in average.

DNA samples for the rare KEL genotypes KEL*6/6 and KEL*17/17 were supplied by S. Lee (New York Blood Center, New York CC). DNA samples of the LU*14/14, KEL*6/6, DI*1/1 and CO*2/2, were given by G. Daniels (International Blood Group Reference Laboratory, Bristol). DNA MNS -3, -4 was sent by Masja de Haas (Saquin, Amsterdam). A DHARSHI cell line homozygous for IN*2/2 was provided by M. Telen (Duke University, North Carolina).

### Phenotyping

Serological typing was performed with regulated reagents (DiaMed AG, Cressier; Ortho-Clinical Diagnostics, Neckargmünd; and Medion Diagnostics GmbH, Düdingen) according to the manufacturers recommendations. Rare blood groups were partly typed with patients antibodies of confirmed specificities.

### Multiplex PCR

Oligonucleotide primer pairs for the determination of the 36 listed polymorphisms are displayed in Table 1. The multiplex PCR assay is organized in six reaction mixes containing five to seven primer pairs (Table 2). The PCR products ranged from 209 to 713 bp. The oligonucleotide mixes were aliquoted in ready-to-use concentrations on 96-well PCR reaction plates. The loaded plates were stored at -20°C.

The PCR reaction was performed using GoTaq kit (Promega, Mannheim). Each reaction tube contained 0,75 U Taq, 3 µL of 5x PCR buffer, 2,5 µL of 25 mM MgCl₂ solution (4,17 nmol/µL), 0,33 mM deoxynucleoside triphosphates (Applichem, Darmstadt), oligonucleotide primers (Table 2) and 35 µg of gDNA to a final reaction volume of 15 µL.

A GeneAmp® PCR System 9700 (Applied Biosystems) was used for the PCR. The temperature profile started with five minutes at 94°C, followed by six cycles at 94°C for 30 seconds, 67°C for 40 seconds (touch down for 0,5°C per cycle) and 72°C for 50 seconds. Subsequently 27 cycles at 94°C for 30 seconds, 64°C for 40 seconds and 72°C for 50 seconds were added. The protocol ended with a final step at 72°C for two minutes.

### Agarose gel electrophoresis

The PCR products were analyzed on 1,5 % agarose gels. The electrophoresis results are shown in Figures 1-6.

**Table 1: PCR primers used in the 36 genotype multiplex-PCR (SEQ ID NO:1-72)**

| Genotype | Primer sequence | Reverse primer sequence |
|---|---|---|
| MNS1 | CAGCATCAAGTACCACTGGT | TTCAGAGGCAAGAATTCCTCCA |
| MNS2 | TCAGCATTAAGTACCACTGAG | TTCAGAGGCAAGAATTCCTCCA |
| MNS3 | CGATGGACAAGTTGTCCCA | GGAGTAATGGCTCCATATGCC |
| MNS4 | CGATGGACAAGTTGTCCCG | GGAGTAATGGCTCCATATGCC |
| LU1 | CGGGAGCTCGCCCgCA | GACAGTGTCCCTTTGTTGGGG |
| LU2 | CGGGAGCTCGCCCgCG | GACAGTGTCCCTTTGTTGGGG |
| LU8 | CTCTCCCAGAGGGCTACAT | GCTTGTGCGACCAATTGAGG |
| LU14 | CTCTCCCAGAGGGCTACAA | GCTTGTGCGACCAATTGAGG |
| KEL1 | ACTCATCAGAAGTCTCAGCA | CTAGAGGGTGGGTCTTCTTCC |
| KEL2 | CTCATCAGAAGTCTCAGCG | CTAGAGGGTGGGTCTTCTTCC |
| KEL3 | TGTCAATCTCCATCACTTCAT | CTGCCCGCACAGGTGGC |
| KEL4 | CAATCTCCATCACTTCACG | CTGCCCGCACAGGTGGC |
| KEL6 | GCCTGGGGGCTGCCC | CTTGCTCACTGGTTCTGC |
| KEL7 | GGGGGCTGCCT | CTTGCTCACTGGTTCTGC |
| KEL11 | GAGCTGGTCGATAGTGA | CTCTTCTTCTCATGCCCCTC |
| KEL17 | AGCTGGTCGATAGTGG | CTCTTCTTCTCATGCCCCTC |
| KEL21 | CAATCTCCATCACTTCACA | CTGCCCGCACAGGTGGC |
| FY1 | CAGCTGCTTCCAGGTTGGgAC | CTCATTAGTCCTTGGCTCTTAT |
| FY2 | CAGCTGCTTCCAGGTTGGgAT | TCATTAGTCCTTGGCTCTTAT |
| FY0 | CTCATTAGTCCTTGGCTCTTAC | CAGCTGCTTCCAGGTTGGgAT |
| FYX | GCTTTTCAGACCTCTCTTCT | CAAATTCCCACAGTGAGC |
| FY5 | GCTTTTCAGACCTCTCTTCC | CAAATTCCCACAGTGAGC |
| JK1 | GTCTTTCAGCCCCATTTGcGG | CCAAGGCCAAGTGTCAGTGC |
| JK2 | AGTCTTTCAGCCCCATTTGcGA | CCAAGGCCAAGTGTCAGTGC |
| DI1 | GGGCCAGGGAGGCCA | TGGCTCCATATGGTGCCTG |
| DI2 | CCAGGGAGGCCG | TGGCTCCATATGGTGCCTG |
| DI3 | CATCATCCAGATGGGAAACTT | GGACTACCTCTACCCCATCC |
| DI4 | ATCATCCAGATGGGAAACTC | GGACTACCTCTACCCCATCC |
| YT1 | CTCATCAACGCGGGAGACTaCC | GAGCCAGAGAGATGAACAGTT |
| YT2 | CTCATCAACGCGGGAGACTaCA | GAGCCAGAGAGATGAACAGTT |
| DO1 | ATTCGATTTGGCCAATTCCTT | TGACCTCAACTGCAACCAGTT |
| DO2 | ATTCGATTTGGCCAATTCCTC | GACCTCAACTGCAACCAGTC |
| CO1 | GAACAACCAGACGGC | CTGAGAGGATGGCGGTGG |
| CO2 | GGGAACAACCAGACGGT | CTGAGAGGATGGCGGTGG |
| IN1 | GTCGCTACAGCATCTCTCC | CCATTCAGCTGTGGGAAAGGAGC |
| IN2 | CGCTACAGCATCTCTCG | CCATTCAGCTGTGGGAAAGGAGC |

| | | |
|---|---|---|
| * Nucleotides written in small letters mark mismatches to the complementary genomic DNA sequence | | |

**Table 2: PCR reaction mixes and product sizes**

| Reaction mix | Genotype | Product size (nt) | Primer conc. (nM1) |
|---|---|---|---|
| 1 | KEL21 | 623 | 1 |
| | DI4 | 538 | 1 |
| | DI1 | 460 | 1 |
| | LU2 | 351 | 1 |
| | FYX | 276 | 1 |
| | IN2 | 209 | 1 |
| 2 | FY0 | 713 | 1 |
| | DI3 | 539 | 1 |
| | DI2 | 457 | 1 |
| | LU1 | 351 | 1 |
| | FY5 | 276 | 1 |
| | IN1 | 211 | 1 |
| 3 | KEL11 | 522 | 1 |
| | KEL6 | 422 | 1 |
| | LU8 | 364 | 1 |
| | YT2 | 300 | 1 |
| | CO1 | 221 | 1 |
| 4 | KEL17 | 521 | 1 |
| | KEL7 | 418 | 1 |
| | LU14 | 364 | 1 |
| | YT 1 | 300 | 1 |
| | CO2 | 223 | 1 |
| 5 | FY1 | 713 | 2 |
| | KEL4 | 623 | 2 |
| | JK1 | 528 | 2 |
| | MNS4 | 397 | 1 |
| | KEL1 | 322 | 1 |
| | MNS1 | 259 | 1 |
| | DO1 | 210 | 1 |
| 6 | FY2 | 712 | 2 |
| | KEL3 | 625 | 2 |
| | JK2 | 529 | 2 |
| | MNS3 | 397 | 1 |
| | KEL2 | 321 | 1 |
| | MNS2 | 260 | 1 |
| | DO2 | 209 | 1 |

As can be seen in the figures, the reaction mixes, particularly mixes 1 to 4, contain regular bands of high frequency antigens. In between or above or below these bands some low or average frequency antigens (<99% prevalence) are included, e.g., Fig. 3: YT2. The high frequency antigens are also used as internal controls.

### Control samples results (Table 3)

For validation of the multiplex-PCR-assay 370 blood samples where serologically typed for the antigens MNS1, MNS2, MNS3, MNS4, LU1, LU2, KEL1, KEL2, KEL3, FY1, FY2, JK1, JK2, using regulated reagents. Additionally, special samples of HFA negative phenotypes or null-types were used for validation of the PCR assay. Two U negative samples (MNS -2,-3) were included. Eight LU 1,-2 samples, 99 KEL 1,-2 samples as well as one KEL 3,-4 sample were also used. Some samples typed for KEL6, KEL7, KEL11, KEL17, DI1, DI2, DI3, DI4, YT1, YT2, DO1, DO2, CO1, CO2, IN1, IN2 were included. Different values in count of antithetical antigens is caused by limited availability of serological reagents. For LU8, LU14, KEL21, FYX and FY5 no control samples could be provided.

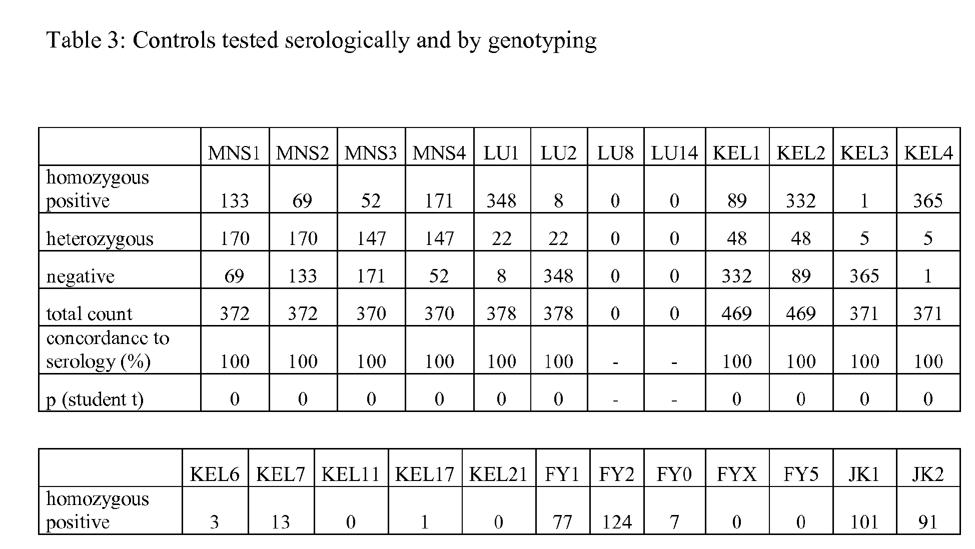

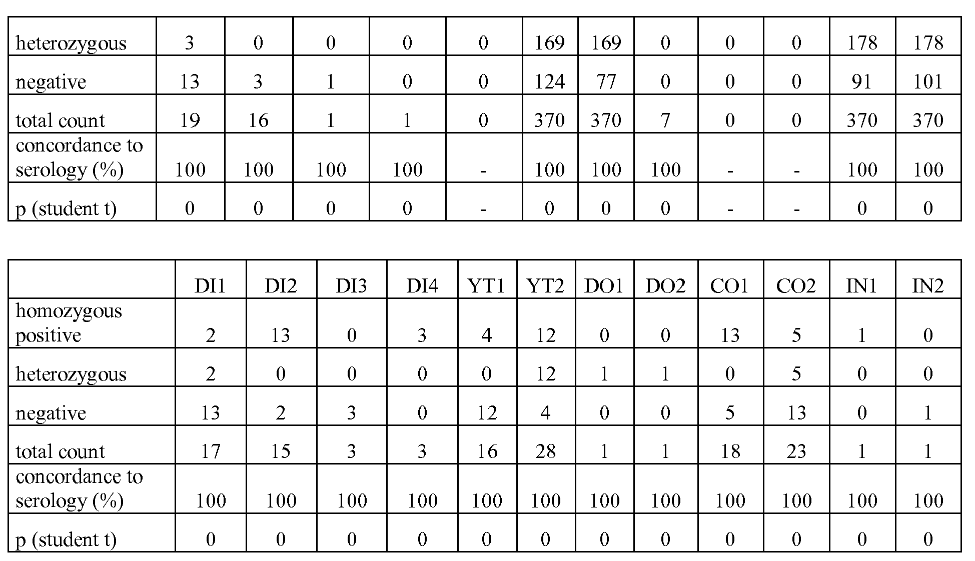

### Findings of HFA negative donors

The genotyping of 6000 randomly selected samples provided 215 988 acceptable results. In 0,014% of the reactions the initial result was indeterminable and a rerun had to be applied. In twelve cases the result remained indeterminable.
57 of the samples tested were found to be lacking a HFA. If serological reagents were available the HFA-negativity was confirmed by serology. In all cases there was concordance between serological testing and the PCR result.

As a result of the screening, nine LU2 negative donors were found, as well as five LU8 negative, five KEL2 negative, one KEL4 negative, 24 YT1 negative and eleven CO1 negative individuals.

### Example 2:

The multiplex-PCR assay was performed as in Example 1, with the exception that a total of 64 RBC surface antigenes were used.

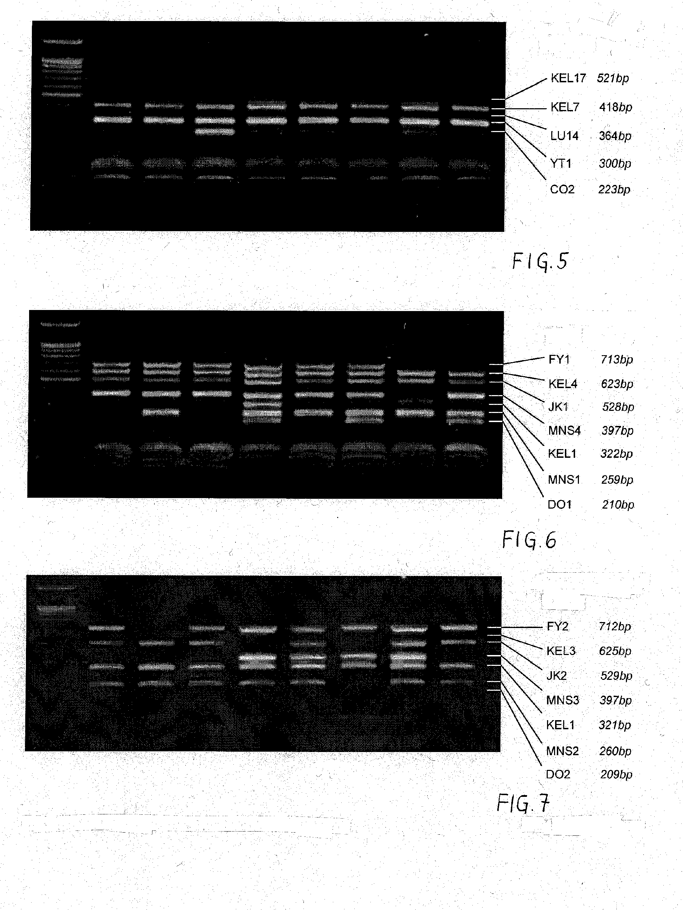

### The electrophoresis results are shown in Figures 7-18.

### Example 3:

The multiplex-PCR assay was performed as in Example 1, with the exception that only platelet surface antigenes were used.

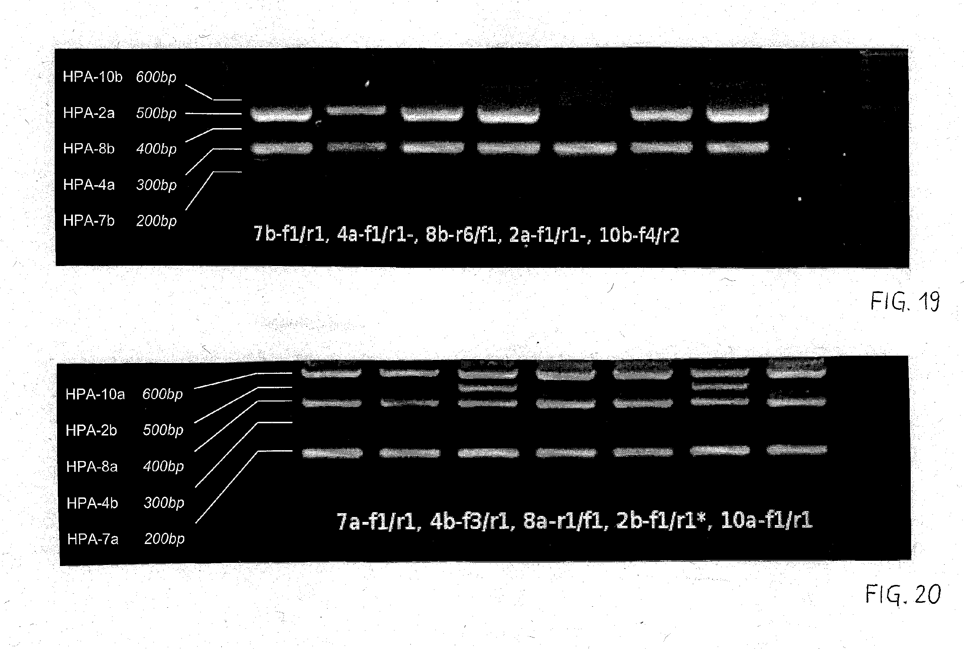

### The electrophoresis results are shown in Figures 19-26.

The examples show that genotyping is a feasible alternative to serological typing. It can provide reliable results and is comparatively inexpensive.

The setup of the inventive assay is clearly arranged. The balanced amplificons' length combined with alternating high and low frequency bands in each reaction mix allows easy examination of the results. Each reaction mix contains four to seven PCR reactions. Most mixes include two or three high incidence SNPs (single nucleotide polymorphisms). These high incidence amplificates have the function of internal controls for the PCR reaction mix.

The inventive assay is appropriate to high-throughput testing. The setup can be done continuously in 96-well microplate format. It may be done either manually or on automated platforms. Even manually approximately 100 individuals (equivalent to 3600 genotypes) can be typed per day.

In comparison to serological typing the multiplex-PCR assay acording to the invention which was set up on a conventional qualitative PCR platform is less costly. The PCR-assay is also very useful in patient typing. The PCR can be applied for antigen-typing in individuals with a positive DAT. Furthermore it can be used in patients with anti-HFA for exclusion of negativity in one of the antigens included in the assay.

### References

Heddle NM, Klama L, Frassetto R, O'Hoski P, Leaman B. A retrospective study to determine the risk of red cell alloimmunisazion and transfusion during pregnancy. Transfusion. 1994 Apr; 34(4):360-1.
Aygun B, Padmanabhan S, Paley C, Chandrasekaran V. Clinical significance of RBC alloantibodies and autoantibodies in sickle cell patients who received transfusions. Transfusion. 2002 Jan; 42(1):37-43.
Ameen R, Al-Shemmari S, Al-Humood S, Chowdhury RI, Al-Eyaadi O, Al-Bashir A. RBC alloimmunization and autoimmunization among transfusion-dependent Arab thalassemia patients.Transfusion. 2003 Nov; 43(11):1604-10.)
Castro O, Sandler SG Houston-Yu P, Rana S. Predicting the effect of transfusing only phenotype-matched RBCs to patients with sickle cell disease: theoretical and practical implications. Transfusion 2002 Jun;42(6):684-90.
Seltsam A, Wagner FF, Salama A, Flegel WA. Antibodies to high-frequency antigens may decrease the quality of transfusion support: an observational study. Transfusion. 2003 Nov;43(11):1563-6.
Blumenfeld OO, Patnaik SK. Allelic genes of blood group antigens: a source of human mutations and cSNPs documented in the Blood Group Antigen Gene Mutation Database. Human Mutation. 2004 Jan; 23(1):8-16.
GYPA-Gene the MNS1 (M), MNS2 (N) polymorphisms GYPB MNS3 (S), MNS4 (s) polymorphisms
Kudo S, Onda M, Fukuda M. Characterization of glycophorin A transcripts: control by the common erythroid-specific promoter and alternative usage of different polyadenylation signals. J Biochem 1994; 116(1):183-92
Siebert PD, Fukuda M. Molecular cloning of a human glycophorin B cDNA: nucleotide sequence and genomic relationship to glycophorin A. Proc Natl Acad Sci U S A 1988; 85(2):421.
LU-Gene LU1 (Lua) and LU2 (Lub)
Parsons SF, Mallinson G, Holmes CH, Houlihan JM, Simpson KL, Mawby WJ, Spurr NK, Warne D, Barclay AN, Anstee DJ. The Lutheran blood group glycoprotein, another member of the immunoglobulin superfamily, is widely expressed in human tissues and is developmentally regulated in human liver. Proc Natl Acad Sci U S A 1995; 92(12):5496-500.
In the KEL-Gene the KEL1(Kell), KEL2 (chellano); KEL3 (Kpa), KEL4 (Kpb), KEL 21 (Kpc); KEL6 (Jsa), KEL7 (Jsb); KEL11 (Cote), KEL17 (Wka) polymorphisms [45,46] were integrated in the test.
Lee S, Wu X, Reid M, Zelinski T, Redman C. Molecular basis of the Kell (K1) phenotype. Blood 1995; 85:912-16.
Lee S. Molecular basis of the Kell blood group phenotypes. Vox Sang 1998; 74:58.
FY
Chaudhuri A, Polyakova J, Zbrzenna V, Williams K, Gulati S, Pogo AO. Cloning of glycoprotein D cDNA, which encodes the major subunit of the Duffy blood group system and the receptor for the Plasmodium vivax malaria parasite. Proc Natl Acad Sci U S A 1993; 90(22):10793-7.
Fy0
Tournamile C, Colin Y, Catron JP, Le Van Kim C. Disruption of a GATA motif in the Duffy gene promoter abolishes erythroid gene expression in Duffy-negative individuals. Nat Gen 1995; 10(2):224-8.
Fyx
Olsson ML, Smythe JS, Hansson C et al. The Fyx-phenotype is associated with a missence mutation in the Fyb allele predicting Arg89Cys in the Duffy glycoprotein. Br J Haematol 1998; 103:1184-91
Touramille C, Lee Van Kim C, Gane P, Le Pennec PY, Roubinet F, Catron JP, Colin Y. Arg89Cys substitution results in very low membrane expression of the Duffy antigen/receptor for chemokines in Fy(x) individuals. Blood 1998; 92(6):2147-56.
Li J, Iwamoto S, Sugimoto N, Okuda H, Kajii E. Dinucleotide repeat in the 3'flanking region provides a clue to the molecular evolution of the Duffy gene. Hum Genet 1997;99:573-7.
JK
Olives B, Merriman M, Bailly P et al. The molecular basis of the Kidd blood group polymorphism and its lack of association with type 1 diabetes susceptibility. Hum Mol Gen 1997;6:1017-20
DI
Bruce LJ, Anstee DJ, Spring FA, Tanner MJA. Band 3 Memphis variant II: altered stilbene disulfonate binding and the Diego (Dia) blood group antigen are associated with the human erythrocyte band 3 mutation Pro854→Leu. J Biol Chem 1994;269:16155-8
Bruce LJ et al. Changes in the blood group Wright antigens are associated with a mutation at amino acid 658 in human erythrocyte band 3: a site of interaction between band 3 and glycophorin A under certain conditions. Blood 1995;85:541-7
DO
Gubin AN, Njoroge JM, Wojda U et al. Identifikation of the Dombrock blood group glycoprotein as a polymorphic member of the ADP-ribosyltransferase gene family. Blood 2000;96:2621-7.
CO
Smith BL, Preston GM, Spring F, Anstee DJ, Agre P. Human red cell Aquaporin CHIP.I. Molecular charakterization of ABH and Colton blood group antigens. J Clin Invest 1994;94:1043-9
YT
Bartels CF, Zelinski T, Lockeridge O. Mutation at codon 322 in human acetylcholinesterase (ACHE) gene accounts for YT blood group polymorphism. Am J Hum Genet 1993;52:928-36
IN
Telen MJ, Udani M, Washington MK et al. A blood group-related polymorphism of CD44 abolishes a hyaluronan-binding consensus sequence without preventing hyaluronan binding. J Biol Chem 1996;271:7147-53

## Claims

1. A method for genotyping blood samples for red blood cell and/or platelet surface antigens containing antigens of ≥99% prevalence and antigens of <99% prevalence in the population, the method comprising the steps of:
(a) extracting DNA from a blood sample,
(b) subjecting the DNA to multiplex PCR, targeting at least three red blood and/or platelet surface antigen genotypes in each reaction mix of at least two reaction mixes,
(c) subjecting amplification products to analysis by electrophoresis, and
(d) comparing the electrophoresis bands obtained in step (c) with bands of positive controls of the red blood cell and/or platelet surface antigens, with identical bands indicating the presence of the red blood cell and/or platelet surface antigens in the sample,
wherein in each PCR reaction mix targets of antigens of ≥99% and <99% prevalence are combined such that alternating bands of antigens of ≥99% prevalence and of <99% prevalence are obtained in step (c).

2. The method according to claim 1, wherein, in step (c), at least 5, preferably 5, red blood and/or platelet surface antigen genotypes are targeted in each reaction mix.

3. The method according to claim 1 or 2, wherein agarose gel electrophoresis or capillary electrophoresis is used in step (d).

4. The method according to any of claims 1 to 3, wherein the red blood cell and/or platelet surface antigens are selected from MNS1, MNS2, MNS3, MNS4, LU1, LU2, LU8, LU14, KEL1, KEL2, KEL3, KEL4, KEL6, KEL7, KEL11, KEL17, KEL21, FY1, FY2, FY0, FYX, FY5, JK1, JK2, DI1, DI2, DI3, DI4, YT1, YT2, DO1, DO2, CO1, CO2, IN1, IN2, LW5, LW6, RHCw, RHC, RHc, RHD, RHE, RHe, CROM1, CROM2, CROM5, CROM6, KN1, KN3, KN4, GE2 YUS, GE3 GE, GE leach PL, GE leach LN, C4A 3258A, C4A (3567A), C4A (3670C), C4B 3258G, C4B (3567G), C4B 3670G, LE1 200T, LE2 428G, LE2 571C, HPA-10a, HPA-10b, HPA-2a, HPA-2b, HPA-8a, HPA-8b, HPA-4a, HPA-4b, HPA-7a, HPA-7b, HPA-14a, HPA-14b, HPA-3a, HPA-3b, HPA-1a, HPA-1b, HPA-6b, HPA-15a, HPA-15b, HPA-11a, HPA-11b, HPA-13a, HPA-13b, HPA-17a, HPA-17b, HPA-5a, HPA-5b, HPA-16a and HPA-16b.

5. The method according to any of claims 1 to 4, wherein only red blood cell surface antigens are targeted in step (c).

6. The method according to any of claims 1 to 4, wherein only platelet surface antigens are targeted in step (c).

7. The method according to claim 5, wherein the at least two reaction mixes are selected from:
Mix 1: KEL21, DI4, DI1, LU2, FYX, IN2
Mix 2: FY0, DI3, DI2, LU1, FY5, IN1
Mix 3: KEL11, KEL6, LU8, YT2, CO1
Mix 4: KEL17, KEL7, LU14, YT1, CO2
Mix 5: FY1, KEL4, JK1, MNS4, KEL1, MNS1, DO1
Mix 6: FY2, KEL3, JK2, MNS3, KEL2, MNS2, DO2
Mix 7: KEL4, KEL1, LW5, RHCw, IN2, RHD
Mix 8: KEL3, CROM1, LW6, KEL2, IN1
Mix 9: GE leach PL, JK1, CROM6, RHC, KN1
Mix 10: GE leach LN, JK2, C4A (3567A), CROM5, RHc, KN3
Mix 11: LE1 200T, C4B 3670G, CROM2, RHE, KN4
Mix 12: LE2 571C, C4A (3670C), C4B (3567G), RHe
Mix 13: FY1, KEL11, KEL6, LU8, YT2, CO1
Mix 14: FY2, KEL17, KEL7, LU14, YT1, CO2
Mix 15: LE2 428G, KEL21, DI4, DI1, LU2, FYX, DO1
Mix 16: FY0, DI3, DI2, LU1, FY5, DO2
Mix 17: GE2 YUS, MNS4, C4A 3258A, MNS1
Mix 18: GE3 GE, MNS3, C4B 3258G, MNS2

8. The method according to claim 4 or 7, wherein the multiplex PCR is performed using one or more of the following primer pairs: SEQ ID NO:1 and SEQ ID NO:2; SEQ ID NO:3 and SEQ ID NO:4; SEQ ID NO:5 and SEQ ID NO:6; SEQ ID NO:7 and SEQ ID NO:8; SEQ ID NO:9 and SEQ ID NO:10; SEQ ID NO: 11 and SEQ ID NO:12; SEQ ID NO:13 and SEQ ID NO:14; SEQ ID NO:15 and SEQ ID NO:16; SEQ ID NO:17 and SEQ ID NO:18; SEQ ID NO:19 and SEQ ID NO:20; SEQ ID NO:21 and SEQ ID NO:22; SEQ ID NO:23 and SEQ ID NO:24; SEQ ID NO:25 and SEQ ID NO:26; SEQ ID NO:27 and SEQ ID NO:28; SEQ ID NO:29 and SEQ ID NO:30; SEQ ID NO:31 and SEQ ID NO:32; SEQ ID NO:33 and SEQ ID NO:34; SEQ ID NO:35 and SEQ ID NO:36, SEQ ID NO:37 and SEQ ID NO:38; SEQ ID NO:39 and SEQ ID NO:40; SEQ ID NO:41 and SEQ ID NO:42; SEQ ID NO:43 and SEQ ID NO:44; SEQ ID NO:45 and SEQ ID NO:46; SEQ ID NO:47 and SEQ ID NO:48; SEQ ID NO:49 and SEQ ID NO:50; SEQ ID NO:51 and SEQ ID NO:52; SEQ ID NO:53 and SEQ ID NO:54; SEQ ID NO:55 and SEQ ID NO:56; SEQ ID NO:57 and SEQ ID NO:58; SEQ ID NO:59 and SEQ ID NO:60; SEQ ID NO:61 and SEQ ID NO:62; SEQ ID NO:63 and SEQ ID NO:64; SEQ ID NO:65 and SEQ ID NO:66; SEQ ID NO:67 and SEQ ID NO:68; SEQ ID NO:69 and SEQ ID NO:70; and/or SEQ ID NO:71 and SEQ ID NO:72.

9. The method according to claim 6, wherein the at least two reaction mixes are selected from:
Mix 1: HPA-10b, HPA-2a, HPA-8b, HPA-4a, HPA-7b
Mix 2: HPA-10a, HPA-2b, HPA-8a, HPA-4b, HPA-7a
Mix 3: HPA-14b, HPA-3a, HPA-1a, HPA-6b
Mix 4: HPA-14a, HPA-3b, HPA-1b
Mix 5: HPA-14b, HPA-15a, HPA-11b, HPA-4a
Mix 6: HPA-14a, HPA-15b, HPA-11a, HPA-4b
Mix 7: HPA-13b, HPA-2a, HPA-17b, HPA-5a, HPA-16b
Mix 8: HPA-13a, HPA-2b, HPA-17a, HPA-5b, HPA-16a

10. A multiplex PCR kit comprising primer pairs for amplifying red blood cell and/or platelet surface antigens selected from MNS1, MNS2, MNS3, MNS4, LU1, LU2, LU8, LU14, KEL1, KEL2, KEL3, KEL4, KEL6, KEL7, KEL11, KEL17, KEL21, FY1, FY2, FY0, FYX, FY5, JK1, JK2, DI1, DI2, DI3, DI4, YT1, YT2, DO1, DO2, CO1, CO2, IN1, IN2, HPA-10a, HPA-10b, HPA-2a, HPA-2b, HPA-8a, HPA-8b, HPA-4a, HPA-4b, HPA-7a, HPA-7b, HPA-14a, HPA-14b, HPA-3a, HPA-3b, HPA-1a, HPA-1b, HPA-6b, HPA-15a, HPA-15b, HPA-11a, HPA-11b, HPA-13a, HPA-13b, HPA-17a, HPA-17b, HPA-5a, HPA-5b, HPA-16a and HPA-16b.

11. The multiplex PCR kit according to claim 10, wherein SEQ ID NO: 1 and SEQ ID NO:2; SEQ ID NO:3 and SEQ ID NO:4; SEQ ID NO:5 and SEQ ID NO:6; SEQ ID NO:7 and SEQ ID NO:8; SEQ ID NO:9 and SEQ ID NO:10; SEQ ID NO: 1 and SEQ ID NO:12; SEQ ID NO:13 and SEQ ID NO:14; SEQ ID NO:15 and SEQ ID NO:16; SEQ ID NO:17 and SEQ ID NO:18; SEQ ID NO:19 and SEQ ID NO:20; SEQ ID NO:21 and SEQ ID NO:22; SEQ ID NO:23 and SEQ ID NO:24; SEQ ID NO:25 and SEQ ID NO:26; SEQ ID NO:27 and SEQ ID NO:28; SEQ ID NO:29 and SEQ ID NO:30; SEQ ID NO:31 and SEQ ID NO:32; SEQ ID NO:33 and SEQ ID NO:34; SEQ ID NO:35 and SEQ ID NO:36, SEQ ID NO:37 and SEQ ID NO:38; SEQ ID NO:39 and SEQ ID NO:40; SEQ ID NO:41 and SEQ ID NO:42; SEQ ID NO:43 and SEQ ID NO:44; SEQ ID NO:45 and SEQ ID NO:46; SEQ ID NO:47 and SEQ ID NO:48; SEQ ID NO:49 and SEQ ID NO:50; SEQ ID NO:51 and SEQ ID NO:52; SEQ ID NO:53 and SEQ ID NO:54; SEQ ID NO:55 and SEQ ID NO:56; SEQ ID NO:57 and SEQ ID NO:58; SEQ ID NO:59 and SEQ ID NO:60; SEQ ID NO:61 and SEQ ID NO:62; SEQ ID NO:63 and SEQ ID NO:64; SEQ ID NO:65 and SEQ ID NO:66; SEQ ID NO:67 and SEQ ID NO:68; SEQ ID NO:69 and SEQ ID NO:70; and/or SEQ ID NO:71 and SEQ ID NO:72 are used as said primer pairs.

12. The multiplex PCR kit according to claim 10 or 11, further comprising a buffer, DNA polymerase, positive and/or negative controls.
